# EUROPEAN PATENT SPECIFICATION

(11) **EP 3 876 823 B1**
(45) Date of publication and mention of the grant of the patent: **03.04.2024**
(21) Application number: 19798414.9
(22) Date of filing: 05.11.2019
(51) Int. Cl.: A61B 5/389, A61B 5/00, A61B 9/00, A61B 5/11

(54) **STRIKER UNIT FOR POSTURAL ANALYSIS**
SCHLAGEINHEIT FÜR POSTURALE ANALYSE
UNITÉ DE PERCUSSION POUR ANALYSE POSTURALE

(30) Priority: 05.11.2018 IT 201800010030
(43) Date of publication of application: 15.09.2021
(73) Proprietor: Politecnico Di Torino, 10129 Torino (IT); Universita' Degli Studi di Torino, 10124 Torino (IT)
(72) Inventor: FERRARESI, Carlo, 10129 Torino (IT); FRANCO, Walter, 10129 Torino (IT); MAFFIODO, Daniela, 10129 Torino (IT); DE BENEDICTIS, Carlo, 10129 Torino (IT); DVIR, Zeevi, 10129 Torino (IT); ROATTA, Silvestro, 10124 Torino (IT)
(74) Representative: Mola, Edoardo
(86) International application number: PCT/IB2019/059499
(87) International publication number: WO 2020/095204

(56) References cited:
- EP-A2- 0 534 503
- WO-A1-2018/152642
- GB-A- 2 168 489

## Description

### TECHNICAL FIELD

The present invention refers to a unit for imparting mechanical perturbations to an individual. The individual is monitored during the perturbation/ s in order to collect and analyze data concerning his postural reaction to the perturbation.

### STATE OF THE ART

The ability to maintain upright standing and/or motion is executed by central and peripheral postural / balance mechanisms and is a fundamental and critical neuro-motor function that enables human beings, along the full spectrum of life, to function and behave normally and efficiently. Disturbances to the normal functioning of postural mechanisms are well known, particularly in patients suffering from neurological disorders, but they also constitute a normal variant of old age, expressed by a higher propensity to falling on the ground in particular as a consequence of an unexpected perturbation.

One way to assess the normal functioning of the postural system is by studying the reaction pattern of the human body to mechanical perturbations while standing, or while walking on either a treadmill or on an instrumented walkway. Perturbations are invariably imparted in the form of some force to the body, either at a fixed anatomical site (e.g. upper trunk) or to the base of support on which the individual is standing, or walking, during the examination.

In particular, the mechanical perturbation is unexpectedly imparted to the individual and various data, including electromyographic, are collected regarding the reaction of the individual to the unexpected perturbation. A parameter that is widely being monitored during perturbation is the center of pressure (CoP), i.e. the point of application of the ground reaction force vector. The location of this point is constantly changing during stance (and walking) but as long as it is located within the area 'enclosed' by the feet, the individual is considered to be in a stable condition or in balance.

Up until now, no standard procedure for imparting perturbations exists for the scope of the subsequent analysis of the center of pressure. Practically all systems that have been described in scientific publications are custom made and lack rigorous testing protocols or reference values. Notably, and specifically with respect to those cases in which a perturbation was applied to the body (and not to the base of support), the force magnitude remained constant irrespective of the individual anthropometry of the subject/patient while the location of the perturbation was limited to one point/area of the body required by the design of the system which imparted the perturbation.

Given the relevance and importance of precise and standard perturbation analysis for scientific and clinical applications, there is therefore a need for a unit that will precisely control the perturbation while taking into account the anthropometric and biomechanical parameters of the individual in order to impart scaled, adaptable and targeted perturbations.

WO-2018-152642 discloses a perturbation device having sensors to detect the motion of the individual after a strike. However, there is no particular mean for real time control of the force imparted during the perturbation, whose value and trend are influenced and modified by the individual reaction, and this causes a substantial lack of information when a perturbation data analysis is performed. GB-A1-2168489 discloses a device for use in a chiropractic activity. Such device comprises a force sensor placed far away from the surface contacting the individual to be treated. Such sensor is not used as feedback device for real time control of the impulse load, but only to measure the applied force, whose value and trend are influenced and modified by the body reaction. The action applied to the individual is slow and having a limited force amplitude. Such teaching is therefore quite far from the needs of a postural analysis. Furthermore, application of the force sensor far from the contact surface implies that the mass of the moving part of the actuator influences the output of the load cell, thus making the sensing relative imprecise, in particular when inertial loads are important such as in the instance of impulse perturbations.

### SCOPES AND SUMMARY OF THE INVENTION

The scope of the present invention is to meet, at least in part, the above mentioned needs.

The scope of the present invention is achieved by a unit for imparting mechanical perturbations to the posture of an individual comprising:
- At least a striker comprising an actuator and a contacting head movable along a rectilinear direction by the actuator so as to impart a mechanical perturbation to the individual;
- A structure supporting the striker on the ground during the operation of imparting the perturbation;
- An electronic control unit programmed to control the contacting head during the perturbation imparting operation of the contacting head;

Wherein
- a load cell is connected to the contacting head to provide the electronic control unit with a force feedback during the perturbation imparting operation about the load applied to the individual; and
- The load cell is applied to the contacting head so as to measure only a component of the force parallel to the axis of translation of the contacting head.

Such unit provides a pre-defined impulse controlled via the load cell. The applicant has found that the magnitude of the sway, a parameter expressed by the length of the trajectory of the CoP was better correlated with the impulse (defined as the integral of the applied force over time), than the peak force of the perturbation. A further improvement to the precision of the analysis relies on the provision of a stable or mobile support or stand to which the striker is attached during provision of the perturbation. Furthermore, one version of the unit of the present invention has a simple layout that, according to an embodiment, may be portable and cost effective, in order to have it applied on a wider clinical scale, e.g. in small clinics or at the home of the individual patient without the need to perform such an examination in a laboratory specializing in postural analysis. The simple layout of the striker is also flexibly applicable to complex supports comprising a plurality of strikers acting along different directions and striking on different areas of the individual, imparted simultaneously or at prescribed time difference, measured in milliseconds. The structure and the load cell provide full control of the location and other characteristics of the perturbation so that the latter can be fully tailored to the specific anthropometry of the individual and the kind of pathology he/she is afflicted with. Furthermore, the load cell constitutes an integral part of the striker and this improves the precision of controlling the perturbation and the correlated feedback, because the kinematic chain between the load cell and the contacting head is very short and possible disturbing parameters, such as stiffness of the support or the like, do not influence the control of the perturbation. Furthermore, a complete postural analysis can be performed without requiring a sensorized / actuated base of support, albeit the latter can be provided in addition to the striker in order to add postural stimuli of a different kind. In particular, when the sensorized / actuated base of support is absent, the perturbation is provided by the striker while the data concerning the response of the individual are collected by other postural data acquisition systems, such as cameras, inertial sensors, electromyography, force plates etc.

When imparting an impulse, it is important to avoid that inertia loads other than the strictly necessary ones are detected by the load cell. Therefore it is important for a precise measure to have the load cell as close as possible to the surface contacting the individual. Furthermore, to increase precision, loads acting transversally to the line of action of the striker, shall not influence the load cell.

According to a preferred embodiment, the control unit is programmed so as to impart a perturbation via the load cell.

According to a preferred embodiment, the unit further comprises data transfer connection with the electronic control unit, at least one of: a sensorized base of support where the individual stands during the action of the striker; at least a kinetic parameter sensing device configured to monitor the motion of the individual during the functioning of the striker; at least a surface electromyography device to monitor muscular latency times and muscular coordination patterns during the perturbation. When the unit comprises one or more of such additional data collectors, the electronic control unit collects the data related to the reactions of the individual to the controlled perturbation provided by the striker. Non limiting examples of possible postural data collection devices comprise: electromyographic sensors, a motion capture apparatus, inertial sensors.

According to a preferred embodiment, the electronic control unit is programmed so as to control the motion of the contacting head according to a predefined kinematic profile before contact with the individual is made, and to control the impulsive force applied to the individual from the instant the contacting head interacts with the body of the individual and up to the termination of such interaction i.e. the full course of the perturbation comprising the combination of an approaching phase and a hitting phase. In order to provide a more stable control in a wider range of perturbing conditions, examples of monitored kinematic variables so as to provide the predefined kinematic profile are the velocity and/or the acceleration of contacting head.

According to a preferred embodiment, the unit comprises a sensor to measure, in use, a distance between the individual and the contact head in a start position and the control unit is programmed so that the kinematic profile is based on such a distance. This ensures that the action of the striker is specifically adapted to the test conditions, e.g. the position of the individual with respect to the striker.

According to a preferred embodiment, the striker is portable and comprises a handle so that a user can hold the striker during impulse imparting and/or a button connected to the control unit so as to start the pre-defined impulse imparting operation of the contacting head.

According to a preferred embodiment, a translation direction is defined by a prismatic guide unit and the load cell is surrounded by the prismatic guide unit and is located in series between the actuator and the contacting head.

### BRIEF DESCRIPTION OF THE DRAWINGS

The invention will be described below on the basis of non-limiting examples shown for explanation purposes in the annexed drawings, which refer respectively:
- Fig. 1 to a flowchart of a perturbation generation and analysis system comprising a striker unit according to the present invention;
- Fig. 2 to a functional sketch of a striker unit according to the present invention;
- Fig. 3 to an exemplar sketch of the striker of fig. 2; and
- Fig. 4 to a preferred embodiment of a contacting head of the striker according to figs 2 and 3.

### DETAILED DESCRIPTION OF THE INVENTION

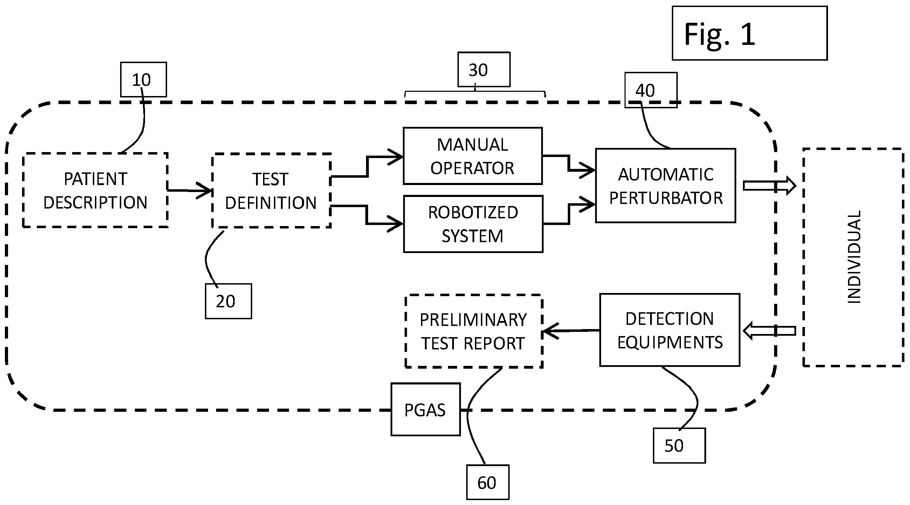

Figure 1 discloses a conceptual sketch of a postural analysis system comprising the steps of describing a patient 10; defining a tailor made postural test 20 based on the step of describing 10; providing a perturbation 30 to a patient or individual either via an operator or via a robotic station through a striker 40 according to the present invention controlled on the basis of the step of defining the postural test 20; collecting postural data e.g. of the sway of the individual, via a postural data detection equipment 50; and providing a preliminary test report 60 based on the automatic and preliminary analysis of the postural data collected via detection equipment 50. As a non-limitative example, detection equipment 50 comprises at least one of: electromyographical sensors, a motion capture apparatus, inertial sensors, a sensorized base of support etc.

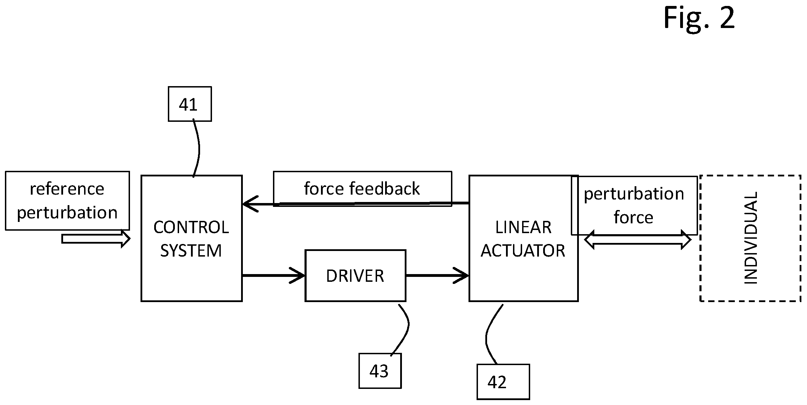

Figure 2 shows a conceptual sketch of striker 40, which comprises an electronic control unit 41 programmed to receive input data about a pre-defined perturbation to be imparted; a linear actuator 42 controlled by the electronic control unit 41 via a driver 43, e.g. a proportional pneumatic or hydraulic valve in case the linear actuator 42 is a pneumatic or hydraulic double-effect piston. In an alternative embodiment, the linear actuator 42 is of the electric type and is driven by a servo-controller 43. Linear actuator 42 imparts a kinetically pre-defined mechanical perturbation to the individual via electronic control unit 43 and is further configured to provide a force feedback to control system 41. In particular, the force feedback is provided via a load cell as explained below in greater detail. Examples of kinetic parameters of the perturbation comprise a force pattern over time during a hitting phase of the perturbation. Preferably, via electronic control unit 43, an approach phase and/or a retraction phase of the perturbation are pre-defined as well, including pre-definition of parameters such as acceleration and/or velocity of linear actuator 42. Preferably, linear actuator 42 retracts swiftly after completion of the hitting phase so as to provide little, if any, interference or disturbance to the countermovement or reaction of the individual.

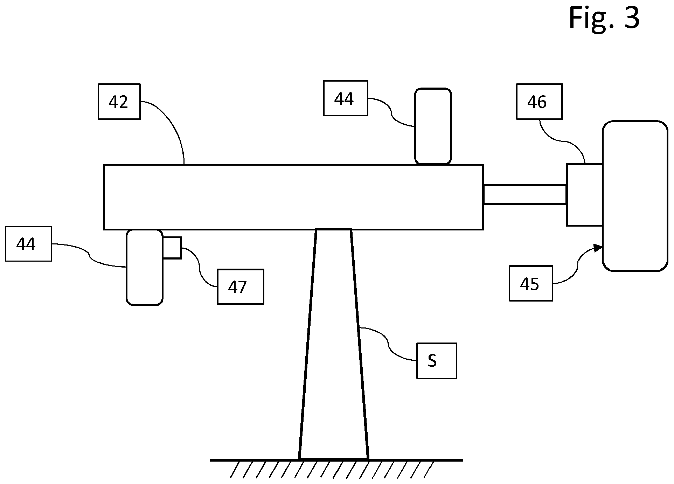

Figure 3 shows a sketch of a striker 40 according to a particularly simple embodiment. In particular, striker 40 according to figure 3 comprises one or more handles 44 so that an operator holds striker 40 during the imparting of a perturbation and provides the necessary inertial reaction, a contacting head 45 carried by linear actuator 42 and movable to contact the individual during imparting of the perturbation, and a load cell 46 placed to measure the load transferred between contacting head 45 and linear actuator 42 along the working direction of the actuator. According to a preferred embodiment, striker 40 also comprises a control button 47 to start a perturbation imparting working cycle of striker 40. Preferably, control button 47 is located close to one handle 44 so that it is possible to press the button with the hand that, at the same time, is holding the handle. For example, button 47 is placed on one handle 44.

Figure 3 also schematically shows a support S so as to define, together with striker 40, a striker unit for imparting mechanical postural perturbations. Support S is configured to provide striker 40 with at least a given position in three-dimensional space. According to the embodiment of figure 3, support S is foldable as a tripod for a camera or the like suitably adapted to the weight of striker 40. Striker 40 is carried by support S via a releasable or detachable hinged joint and the support S is preferably either foldable or collapsible or demountable in a manner similar to tripods for a camera so as to be portable in a handbag. Also striker 40 can be configured to be portable in a handbag.

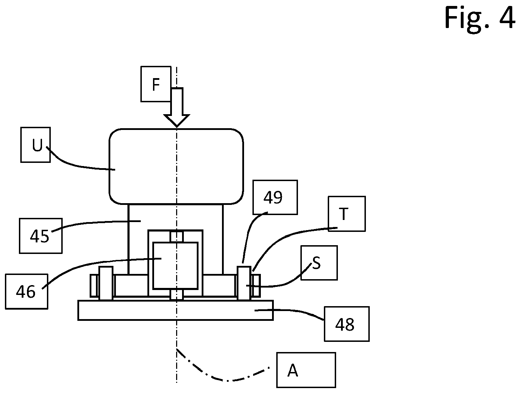

Figure 4 shows an operating embodiment of contacting head 45. Contacting head 45 is preferably rounded and/or convex to avoid sharp wedges in order to impart the perturbation without hurting the individual. For example, contacting head 45 may also comprise an upholstered portion U made of a viscoelastic material, to be more comfortable for the individual. Contacting head may come in different sizes (diameters) to match individual anthropometry as well as the need to target smaller or larger skin areas.

Load cell 46 is preferably located along the motion axis A of linear actuator 42, which coincides with an axis or plane of symmetry of contacting head 45, if any.

In particular, contacting head 45 is carried by linear actuator 42 via a base 48, e.g. a flange, and a prismatic guide 49 between base 48 and contacting head 45 to guide contacting head 45 along a direction parallel to that of axis A. Preferably, prismatic guide 49 comprises one or more projections P surrounding load cell 46 and axially coupled in respective seats T. According to the embodiment in figure 4, projections P are carried by base 48 and seats T are defined by contacting head 45. As shown in figure 4, preferably the load cell 46 is located between contacting head 45 and base 48. Prismatic guide 49 also defines stops to avoid that contacting head 45 is detached from base 48 along a direction from the base to the upholstered portion U and, at the same time, guides in the opposite direction, i.e. the direction of compression of the load cell 46, contacting head 45 during imparting of the perturbation.

Preferably, electronic control unit 41 is programmed to provide, via load cell 46, a mechanical perturbation in the form of an impulse, i.e. the integral of a pre-defined force pattern over time, or in any other form deemed convenient. In order to increase stability of such a control in a wider range of different perturbations, electronic control unit 41 is preferably programmed to control the perturbation also based on velocity and/or acceleration of contacting head 45. In particular, velocity and/or acceleration are pre-defined so as to reach a given value when contacting head 45 first touches the individual. In order to do so, electronic control unit 41 is programmed to receive an input about the distance between contacting head 45 in a given starting position of a relative perturbation imparting working cycle and a contact point on the individual for contacting head 45. Such distance may be measured by detection equipment 50 and, in particular, when the individual is set in a suitable position for the postural examination, and during an approaching phase of the stroke towards the target on the individual and at the instant of the impact.

It is understood that modifications or changes to the striker unit may be provided without departing from the scope of protection as defined in the appended claims. For example, according to other embodiments of the present invention, support S also provides itself with the inertial reaction or sufficient stiffness for rigidly reacting to the mechanical perturbation imparted to the individual. For example, support S may have a portal structure and, may also carry more than one striker 40 in order to impart complex perturbations to the individual and most importantly, may move along a closed (circular, oval) track around the individual, in order to impart the perturbation/ s from different directions.

Furthermore, positioning and orientation of striker 40 on support S may be either manual, as described above, or automated e.g. via actuators or robots having one or more degrees of freedom. Positioning and angular orientation of the striker(s) is preferably performed in a set-up phase of the striker unit, after which the position(s) and orientation(s) remain unchanged during the perturbation imparting. The invention is defined by appended claims 1-12.

## Claims

1. A unit for imparting mechanical perturbations to the posture of an individual comprising:
- At least a striker (40) comprising an actuator (42) and a contacting head (45) movable along a rectilinear direction by the actuator so as to impart a perturbation to a target on the individual;
- A structure (S) supporting the striker on the ground during the operation of imparting the perturbation;
- An electronic control unit (41) programmed to control the contacting head (45) during the perturbation imparting operation;
**characterized in that**
- a load cell (46) is connected to the contacting head (45) to provide the electronic control unit (41) with a force feedback during the perturbation imparting operation about an impulse load applied to the individual; and
- The load cell (46) is applied to the contacting head (45) so as to measure only a component of the impulse perturbation force parallel to an axis (A) of translation of the contacting head (45).

2. The unit according to claim 1, wherein the load cell (46) is located between the linear actuator (42) of the striker and the contacting head (45).

3. The unit according to claims 1 or 2, wherein the electronic control unit (41) is programmed so as to cause the imparting of the perturbation according to a pre-defined kinetic profile over time via the actuator (42) and the load cell (46).

4. The unit according to any of the preceding claims, wherein the control unit (41) is programmed so as to swiftly retract the contacting head (45) to such position that does not disturb a possible countermovement of the tested person after the perturbation.

5. The unit according to any of the preceding claims, further comprising detection
equipment and sensors (50) in data transfer connection with the electronic control unit (41) and configured to detect the relative position between the contacting head (45) and the target during an approach phase of the stroke and the reaction of the individual to the perturbation during and/or after a hitting phase of the stroke.

6. The unit according to any of the preceding claims, wherein the electronic control unit (41) is programmed so as to control the perturbation according to at least a predefined kinematic parameter of the contacting head (45) at least as long as the contacting head (45) is in an approach phase of the stroke wherein it moves towards the individual without contacting the individual.

7. The unit according to claim 5, further comprising a sensor to measure, in use, the distance between the individual and the contact head (45) set in a start position and wherein the control unit (41) is programmed so that the motion of the contacting head (45) while approaching the individual is based on such a distance to reach a pre-defined value of the kinematic parameter at the point where the contacting head (45) touches the individual.

8. The unit according to any of the preceding claims, wherein the striker (40) is portable and comprises at least a handle (44) to hold the actuator (42) during the imparting of the perturbation.

9. The unit according to claim 8, wherein the support (S) is detachable from the striker (40) and is foldable or collapsible or demountable so as to be portable in a handbag.

10. The unit according to any of claim 8 or 9, comprising a control button (47) connected to the electronic control unit (41) so as to start a perturbation imparting working cycle and located so that an operator can press the control button (47) while holding the handle (44).

11. The unit according to any of the preceding claims, comprising a plurality of strikers (40).

12. Test method for a postural analysis of an individual comprising the step of applying a perturbation to the posture of the individual via a unit according to any of the preceding claims.

## Patentansprüche

1. Einheit zur Übertragung von mechanischen Störungen der Haltung einer Person, bestehend aus:
- mindestens einem Schlagbolzen (40), der einen Aktuator (42) und einen Kontaktkopf (45) umfasst, der durch den Aktuator entlang einer geradlinigen Richtung bewegbar ist, um auf die Person eine Störung bis auf einem Ziel auszuüben;
- einer Struktur (S), die den Schlagbolzen während des Vorgangs der Übertragung der Störung auf dem Boden stützt;
- einer elektronischen Steuereinheit (41), die so programmiert ist, dass sie den Kontaktkopf (45) während des Vorgangs der Übertragung der Störung steuert;
**dadurch gekennzeichnet, dass**
- eine Kraftmessdose (46) mit dem Kontaktkopf (45) verbunden ist, um der elektronischen Steuereinheit (41) während des Vorgangs der Übertragung der Störung eine Kraftrückmeldung über eine auf die Person ausgeübte Impulslast zu liefern; und
- die Kraftmessdose (46) wird an den Kontaktkopf (45) angelegt, um nur eine Komponente der Impulsstörungskraft parallel zu einer Translationsachse (A) des Kontaktkopfes (45) zu messen.

2. Einheit nach Anspruch 1, wobei die Kraftmessdose (46) zwischen dem linearen Aktuator (42) des Schlagbolzens und dem Kontaktkopf (45) angeordnet ist.

3. Einheit nach Anspruch 1 oder 2, wobei die elektronische Steuereinheit (41) so programmiert ist, dass sie die Übertragung der Störung gemäß einem vordefinierten kinetischen Profil im Laufe der Zeit über den Aktuator (42) und die Kraftmessdose (46) bewirkt.

4. Einheit nach einem der vorhergehenden Ansprüche, wobei die Steuereinheit (41) so programmiert ist, dass sie den Kontaktkopf (45) schnell in eine solche Position zurückzieht, dass eine mögliche Gegenbewegung der getesteten Person nach der Störung nicht gestört wird.

5. Einheit nach einem der vorhergehenden Ansprüche, ferner umfassend eine Erfassungsausrüstung und Sensoren (50), die in Datenübertragungsverbindung mit der elektronischen Steuereinheit (41) stehen und so konfiguriert sind, dass sie die relative Position zwischen dem Kontaktkopf (45) und dem Ziel während einer Annäherungsphase des Hubes und die Reaktion der Person auf die Störung während und/oder nach einer Kontaktphase des Hubes erfassen.

6. Einheit nach einem der vorhergehenden Ansprüche, wobei die elektronische Steuereinheit (41) so programmiert ist, dass sie die Störung gemäß mindestens einem vordefinierten kinematischen Parameter des Kontaktkopfes (45) mindestens so lange steuert, wie der Kontaktkopf (45) sich in einer Annäherungsphase des Hubes befindet, in der er sich auf die Person zubewegt, ohne die Person zu berühren.

7. Einheit nach Anspruch 5, ferner einen Sensor umfassend, um im Gebrauch den Abstand zwischen der Person und dem Kontaktkopf (45), der sich in einer Startposition befindet, zu messen, und wobei die Steuereinheit (41) so programmiert ist, dass die Bewegung des Kontaktkopfes (45) beim Annähern an die Person basiert auf einem solchen Abstand, so dass ein vordefinierter Wert des kinematischen Parameters an dem Punkt erreicht wird, an dem der Kontaktkopf (45) die Person berührt.

8. Einheit nach einem der vorhergehenden Ansprüche, wobei der Schlagbolzen (40) tragbar ist und mindestens einen Griff (44) umfasst, um den Aktuator (42) während der Ausübung der Störung zu halten.

9. Einheit nach Anspruch 8, wobei die Stütze (S) vom Schlagbolzen (40) abnehmbar und faltbar oder zusammenklappbar oder abnehmbar ist, um in einer Handtasche tragbar zu sein.

10. Einheit nach einem der Ansprüche 8 oder 9, umfassend einen Steuerknopf (47), der mit der elektronischen Steuereinheit (41) verbunden ist, um einen störungserzeugenden Arbeitszyklus zu starten, und der so angeordnet ist, dass ein Bediener den Steuerknopf (47) drücken kann, während er den Griff (44) festhält.

11. Einheit nach einem der vorhergehenden Ansprüche, umfassend mehrere Schlagbolzen (40).

12. Testverfahren für eine Haltungsanalyse einer Person, das den Schritt des Ausübens einer Störung auf die Haltung der Person über eine Einheit nach einem der vorhergehenden Ansprüche umfasst.

## Revendications

1. Unité pour transmettre des perturbations mécaniques à la posture d'un individu comprenant:
- au moins un percuteur (40) comprenant un actionneur (42) et une tête de contact (45) mobile le long d'une direction rectiligne par l'actionneur de manière à conférer une perturbation à une cible sur l'individu;
- une structure (S) supportant le percuteur au sol pendant l'opération de transmission de la perturbation;
- une unité de commande électronique (41) programmée pour contrôler la tête de contact (45) pendant l'opération de transmission de la perturbation;
**caractérisée en ce que**
- une cellule de charge (46) est connectée à la tête de contact (45) pour fournir à l'unité de commande électronique (41) un retour de force pendant l'opération conférant une perturbation autour d'une charge d'impulsion appliquée à l'individu; et
- la cellule de pesée (46) est appliquée à la tête de contact (45) de manière à mesurer uniquement une composante de la force de perturbation d'impulsion parallèle à un axe (A) de translation de la tête de contact (45).

2. Unité selon la revendication 1, dans laquelle la cellule de charge (46) est située entre l'actionneur linéaire (42) du percuteur et la tête de contact (45).

3. Unité selon la revendication 1 ou 2, dans laquelle l'unité électronique de commande (41) est programmée de manière à provoquer l'application de la perturbation selon un profil cinétique prédéfini dans le temps par l'actionneur (42) et la cellule de charge (46) .

4. Unité selon l'une quelconque des revendications précédentes, dans laquelle l'unité de commande (41) est programmée de manière à rétracter rapidement la tête de contact (45) vers une position telle qui ne perturbe pas un éventuel contre-mouvement de la personne testée après la perturbation.

5. Unité selon l'une quelconque des revendications précédentes, comprenant en outre un équipement de détection et des capteurs (50) en connexion de transfert de données avec l'unité de commande électronique (41) et configurés pour détecter la position relative entre la tête de contact (45) et la cible pendant une phase d'approche de la course et une réaction de l'individu à la perturbation pendant et/ou après une phase de frappe de la course.

6. Unité selon l'une quelconque des revendications précédentes, dans laquelle l'unité électronique de commande (41) est programmée de manière à contrôler la perturbation selon au moins un paramètre cinématique prédéfini de la tête de contact (45) au moins aussi longtemps que la tête de contact (45) se trouve dans une phase d'approche de la course dans laquelle elle se dirige vers l'individu sans entrer en contact avec l'individu.

7. Unité selon la revendication 5, comprenant en outre un capteur pour mesurer, en utilisation, la distance entre l'individu et la tête de contact (45) réglée dans une position de départ et dans laquelle l'unité de commande (41) est programmée de telle sorte que le mouvement de la tête de contact (45) lors de l'approche de l'individu est basée sur une telle distance pour atteindre une valeur prédéfinie du paramètre cinématique au point où la tête de contact (45) touche l'individu.

8. Unité selon l'une quelconque des revendications précédentes, dans laquelle le percuteur (40) est portable et comprend au moins une poignée (44) pour maintenir l'actionneur (42) pendant l'application de la perturbation.

9. Unité selon la revendication 8, dans laquelle le support (S) est détachable du percuteur (40) et est pliable ou repliable ou démontable de manière à être portable dans un sac à main.

10. Unité selon l'une quelconque des revendications 8 ou 9, comprenant un bouton de commande (47) connecté à l'unité électronique de commande (41) de manière à démarrer un cycle de travail générant des perturbations et situé de telle sorte qu'un opérateur puisse appuyer sur le bouton de commande (47) tout en tenant la poignée (44).

11. Unité selon l'une quelconque des revendications précédentes, comprenant une pluralité de percuteurs (40) .

12. Procédé d'essai pour une analyse posturale d'un individu comprenant l'étape consistant à appliquer une perturbation à la posture de l'individu par une unité selon l'une quelconque des revendications précédentes.
